# EUROPEAN PATENT SPECIFICATION

(11) **EP 1 295 131 B1**
(45) Date of publication and mention of the grant of the patent: **21.03.2007**
(21) Application number: 01925851.6
(22) Date of filing: 29.04.2001
(51) Int. Cl.: G01N 33/90, G01N 33/52

(54) **PROCESS FOR THE MEASUREMENT OF NON-TRANSFERRIN BOUND IRON**
VERFAHREN ZUR MESSUNG VON DURCH TRANSFERRIN NICHT-GEBUNDENEM EISEN
METHODE AMELIORE DE MESURE DE FER NON LIE A LA TRANSFERRINE

(30) Priority: 30.04.2000 IL 13588400
(43) Date of publication of application: 26.03.2003
(73) Proprietor: YISSUM RESEARCH DEVELOPMENT COMPANY OF THE HEBREW UNIVERSITY OF JERUSALEM, 91390 Jerusalem (IL)
(72) Inventor: Breuer, William, P.O. Box 133 99875 M.P. HaEla (IL); Cabantchik, Yoav Zvi, Har Hadar 90836 (IL)
(74) Representative: Schmitz, Jean-Marie
(86) International application number: PCT/IL2001/000384
(87) International publication number: WO 2001/084161

(56) References cited:
- PETRAT FRANK ET AL: "Determination of the chelatable iron pool of isolated rat hepatocytes by digital fluorescence microscopy using the fluorescent probe, phen green SK." HEPATOLOGY, vol. 29, no. 4, April 1999 (1999-04), pages 1171-1179, XP008007487 ISSN: 0270-9139
- PETRAT FRANK ET AL: "Determination of the chelatable iron pool of single intact cells by laser scanning microscopy." ARCHIVES OF BIOCHEMISTRY AND BIOPHYSICS, vol. 376, no. 1, 1 April 2000 (2000-04-01), pages 74-81, XP002211519 ISSN: 0003-9861
- BREUER WILLIAM ET AL: "Iron acquired from transferrin by K562 cells is delivered into a cytoplasmic pool of chelatable iron(II)." JOURNAL OF BIOLOGICAL CHEMISTRY, vol. 270, no. 41, 1995, pages 24209-24215, XP002211549 ISSN: 0021-9258
- LYTTON S D ET AL: "MONITORING OF IRON-III REMOVAL FROM BIOLOGICAL SOURCES USING A FLUORESCENT SIDEROPHORE" ANALYTICAL BIOCHEMISTRY, vol. 205, no. 2, 1992, pages 326-333, XP002211547 ISSN: 0003-2697
- VAN RENSWOUDE J ET AL: "RECEPTOR MEDIATED ENDOCYTOSIS OF TRANSFERRIN AND THE UPTAKE OF IRON IN K-562 CELLS IDENTIFICATION OF A NONLYSOSOMAL ACIDIC COMPARTMENT" PROCEEDINGS OF THE NATIONAL ACADEMY OF SCIENCES OF THE UNITED STATES, vol. 79, no. 20, 1982, pages 6186-6190, XP002211520 1982 ISSN: 0027-8424
- BREUER WILLIAM ET AL: "Desferrioxamine-chelatable iron, a component of serum non-transferrin-bound iron, used for assessing chelation therapy." BLOOD, vol. 97, no. 3, 1 February 2001 (2001-02-01), pages 792-798, XP002211521 ISSN: 0006-4971
- BREUER WILLIAM ET AL: "A fluorescence-based one-step assay for serum non-transferrin-bound iron." ANALYTICAL BIOCHEMISTRY, vol. 299, no. 2, 15 December 2001 (2001-12-15), pages 194-202, XP002211522 ISSN: 0003-2697

## Description

The present invention concerns a method of determining the content of non-transferring bound iron (NTBI) and/or chelator accessible iron in a sample.

This invention relates to a measure for measuring the level of non-transferrin bound iron (hereinafter "NTBI"), which is relatively simpler and easier to perform than existing methods.

The invention also concerns a reagent for the measurement of non transferrin bound iron.

The presence of non-transferrin-bound iron (NTBI) in the circulation is a pathological phenomenon which occurs in patients with iron-overload conditions. NTBI is absent from healthy individuals where virtually all of the serum iron is bound to the iron-carrier protein transferrin. However, in iron-overloaded individuals, the iron binding capacity of transferrin is overwhelmed, resulting in the adsorption of the excess iron to various proteins and possibly other molecules in the serum. This iron so adsorbed is collectively designated as NTBL

Generally, chronic iron-overload accompanied by NTBI occurs as a result of pathological conditions associated with specific diseases. Illustrative examples of such conditions include: 1) repeated transfusions, which are required by patients with various hemolytic diseases, hemoglobinopathies (among which the most common is thalassemia) or other forms of anemia whose treatment demands blood transfusions and/or infusion of iron (*e.g*. dialysis patients) and 2) an inherited defect causing excess iron absorption, called Hereditary Hemachromatosis. Transient, reversible NTBI can also appear in the circulation of patients undergoing chemotherapy, heart bypass operations and other conditions where large amounts of iron, such as from hemoglobin catabolism, are suddenly released into the circulation. NTBI was also found in dialysis patients who are treated for anemia with erythropoietin and IV iron supplements.

The art has suggested methods for NTBI determination. One methodology was originally developed by Hershko and coworkers [Hershko, H., Graham, G., Bates, G.W., and Rachmilewitz (1978) *British J. Haematol.* 40, 255-263] and later refined by Singh and coworkers [Singh, S., Hider, R.C. and Porter, J.B. (1990) *Anal. Biochem.* 186, 320-323]. In brief, the refined method is as follows:
Step 1. A serum sample (1 ml) is mixed with 80 mM nitrilotriacetic acid (to solubilize the NTBI);
Step 2. The sample is filtered by centrifugation on Centricon filters with a 25 kD molecular weight cut-off;
Step 3. The protein-free filtrate is injected into an HPLC column derivatized with the iron chelator deferriprone (or L1), which forms a stoichiometric coloured complex with iron giving a quantitative value of the amount of iron in the sample.

The three main drawbacks of this method are its cost, its cumbersome nature, which makes it difficult to set up in non-specialized laboratories, and its relatively low throughput efficiency.

A second method [Evans, P.J. and Halliwell, B. (1994) *Methods Enzymol.,* 233, 82-89] employs the antibiotic bleomycin, which combines with NTBI, but not with transferrin-bound iron, to form highly reactive complexes which generate DNA cleavage products. The relative amount of DNA cleavage products is proportional to the amount of input NTBI and is quantified by the thiobarbituric acid test. The drawback of this method is that it tends to overestimate NTBI and may give false positive results.

Co-pending patent publication 11127621 (published on May 23, 2002, filed after the priority date of this application) of the same applicant, describes and claims a method for determining the concentration of a non-bound metal ion - particularly non-transferrin bound iron (NTBI) - in a sample of serum or other biological fluids, comprising the steps of:
a) providing a surface coated with a polymer-conjugated form of a metal chelator - particularly a desferrioxamine (DFO) polymer;
b) bringing said sample into contact with said coated surface, under conditions and for a period of time sufficient to allow the metal ion to be captured by the metal chelator;
c) bringing into contact with said coated surface, after completion of step b) above, a marker conjugated with a moiety that can be captured by the metal chelator, which can be, e.g., the same metal ion the concentration of which it is desired to determine;
d) determining the amount of marker that has been released by the capture of the metal ion by the coated surface; and
e) calculating the concentration of the metal ion in the sample from the concentration of binding sites left available after step b) for capturing the metal ion bound to the marker.

The term "marker" means herein any substance the concentration of which can be precisely determined by any means, particularly by chemical determination. The marker can conveniently be a fluorescent probe. The term "conjugated" means herein any combination in which one end is suitable to combine with the bound chelator, and another end is detectable and functions as a marker. Such a combination may typically be a complex. According to a preferred embodiment of said method the marker is a calcein-iron complex.

While the process of said co-pending publication is highly effective, it has the disadvantage of comprising several discrete steps. These make the process labor-intensive and each additional step decreases the precision of the measurement.

A fluorescent probe, that is composed of a fluorescein molecule attached to a desferrioxamine polymer and will be indicated herein as Fl-DFO, has become commercially available. Molecular Probes, Inc., Eugene, Oregon, USA formerly sold Fl-DFO and will accept orders for it as a custom synthesis by special order.

The use of Fl-DFO meets with a problem which is common to any fluorescent probe: it is sensitive to the environment, that is to say that the fluorescence is affected by color, turbidity, pH, ionic strength, etc. Therefore, any fluorescence detected could be either due to the presence of iron or to some unknown fluorescence-quenching component.

It is therefore a purpose of this invention to overcome the aforesaid problem, and to provide a method for the determination of NTBI, particularly in biological fluids, that permits to compensate for environmental factors and, in general, for any factors not due to the presence of iron in the sample, particularly a sample of body fluid.

It is another purpose to provide such a method that does not require the coating of plates with a polymer-conjugated form of a metal chelator.

It is a further purpose to provide such a method that requires a smaller number of steps than those of the prior art.

It is a still further purpose to provide such a method that achieves the foregoing purposes without any significant loss of sensitivity.

It is a still further purpose to accurately detect NTBI even in serum samples containing near normal levels of apo-Transferrin.

It is a still further purpose is to avoid the interference of endogenous serum apo-Transferrin in the measurement of non-transferrin bound iron.

A particular purpose is to render serum NTBI always directly accessible to the Fe chelator DFO.

Another particular purpose is to provide a process for the preparation of Fluorescein-labeled apo-Transferrin.

Other purposes and advantages of the invention will appear as the description proceeds.

The method according to the present invention is described in claim 1. Preferred embodiments are shown in the sub-claims.

The method according to the invention comprises two stages, each of which comprises subjecting a sample to two measurements for the detection of NTBI, wherein in the first measurement the sample is contacted with a fluorescent probe (which is stoichiometrically quenched by iron) and in the second measurement it is contacted with the same fluorescent probe in the additional presence of a large amount, or excess, of a Fe chelator. By "large amount", or "excess", is meant herein an amount that is at least the amount sufficient, when added to a sample known to contain no NTBI, under the same conditions under which the assay is carried out, to cause maximum fluorescence, viz. the fluorescence corresponding to zero Fe content in the calibration curve. Preferably, the fluorescent probe comprises a fluorescent marker - more preferably fluorescein - combined with a Fe chelator. In an embodiment of the invention, said fluorescent probe is fluoresceinated-deferrioxamine (Fl-DFO), which is capable of direct detection of 0.4 - 7 µM iron concentrations in an ELISA (Enzyme Linked Immunoassay) format. In another, particularly preferred embodiment, said fluorescent probe is fluorescein-labeled apo-Transferrin (Fl-aTf). Also, preferably, the Fe chelator used in the second stage is the same that is part of the fluorescent probe -preferably DFO or Fl-aTf. Transferrin-bound iron does not contribute to the measurements.

The use of Fl-aTf as fluorescent probe is particularly preferred for the following reason. One fundamental technical problem in measuring NTBI has been interference by endogenous serum apo-Transferrin (apo-TF, or Transferrin capable of iron binding). Said apo-Tf is universally found in human sera, except in cases of extreme iron-overload where the Transferrin is 100% iron-saturated. Therefore the detection of NTBI may be rendered more difficult if the serum sample contains nearly normal levels of apo-Tf. The use of Fl-aTf as a probe equalizes the probability that the mobilized NTBI will bind to the detection probe or to apo-Tf in the sample. Fl-aTf is used, in the art, in cell biology research, but its use as a fluorescent iron detector has never been proposed, and such use is an aspect of the invention.

The invention also comprises a most preferred form in which a source of Gallium ions, e.g. a Gallium halide, added to the Fl-aTf probe, is used to block the apo-Tf in the sample. This metal mimics iron and binds to apo-Tf, preventing its reaction with iron. It might be expected that the iron-induced fluorescence change of Fl-aTf would be blocked by Gallium. However, this does not occur, though why is not yet understood. This apparent insensitivity to Gallium gives the Fl-aTf probe an iron-binding advantage over the endogenous aTf, overcoming most of its interference.

The fluorescence obtained in the first measurement of the method of the invention is due to the binding of iron, if any, to the fluorescent probe, and to the possible effect of other, unknown factors in the samples. The fluorescence obtained in the second measurement is due only to said other, unknown factors, since the added excess of Fe chelator removes all the iron from the sample. The ratio of the fluorescent signals obtained in the two conditions indicates whether there is a detectable iron in the sample or not. In principle, if the two signals are equal, this indicates that there is no iron in the sample, while if the second signal is smaller than the first, this means that there is iron in the sample.

It is to noted that serum NTBI is not always directly accessible to DFO. In the aforesaid copending publication 11127621 it is suggested to mobilize (or solubilize) the NTBI, that is not directly DFO-accessible, by means of with nitrilotriacetate. Mobilization of said NTBI is also carried out in an embodiment of this invention, more preferably by means of a mobilizing agent mixture that will be described hereinafter.

Further, this invention comprises a method of preparation of Fl-aTf. The conventional method comprises reacting iron-loaded Tf with Fluorescein Isothiocyanate (FITC). The method of this invention comprises reacting iron-free aTf with 5-4,6-dichlorotriazinyl aminofluorescein (DCTF). As a result, the Fl label is attached to a location on the Tf protein different from that which results from the conventional method and the Fl-aTf is a new product that binds differently to anti-fluorescein antibodies.

### Brief Description of the Drawings

In the drawings:
Fig. 1 is a schematic illustration of an embodiment of the method of the invention;
Fig. 2 is an example of the plot of the ratio of fluorescence of the plate samples generated by addition of reagents A and B *versus* the logarithm of the input [Fe] concentration ;
Fig. 3 depicts the linear range (0-7 µM Fe) of the curve shown in Fig. 2;
Fig. 4 is similar to Fig. 1, but illustrates another embodiment; and
Fig. 5 is similar to Fig. 3, but relates to the embodiment of Fig. 4.

### Detailed Description of Preferred Embodiments

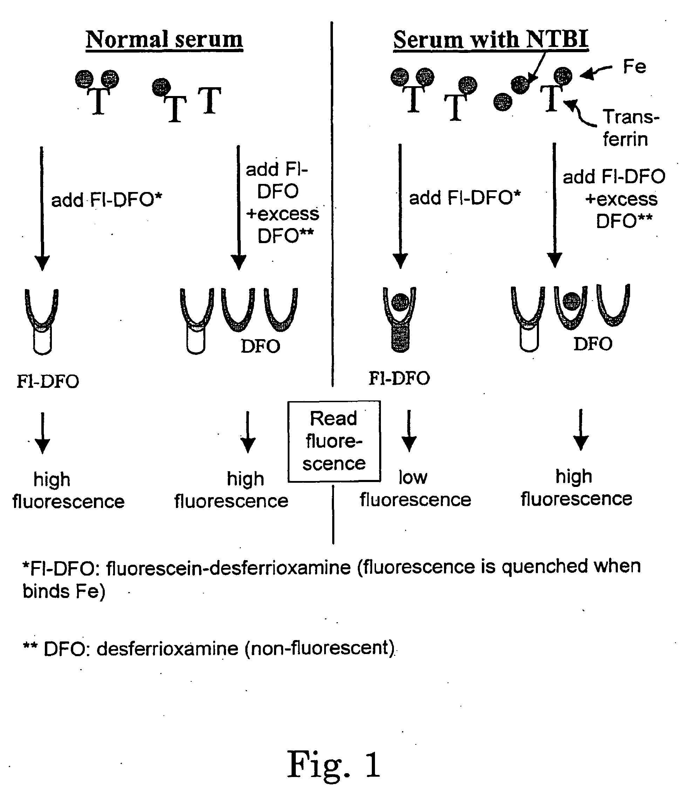

The NTBI assay according to an embodiment of the invention is schematized in Fig. 1. In the first stage of the method of the invention the liquid sample on which the assay is carried out is contacted with a solution of a fluorescent probe, which in this embodiment is preferably Fl-DFO (hereinafter, also "reagent A") and the resulting fluorescence is determined. In the second stage the sample is contacted with the same fluorescent probe Fl-DFO solution, to which however an excess - viz. a large amount, as hereinbefore defined - of a Fe chelator, preferably DFO, has been added (hereinafter also, "reagent B"), and the resulting fluorescence is determined. The left side of Fig. 1 schematizes what happens if the sample does not contain non-transferrin bound iron (NTBI): the same fluorescence is obtained in both measurements, indicating the absence of NTBI. The right side of Fig. 1 schematizes what happens if the sample contains non-transferrin bound iron: different fluorescence values are obtained in the two measurements and the difference between the two values indicates the amount of NTBI in the sample, since the first fluorescence is due to the combined influence on NTBI and other factors, while the second is due to the influence of said other factors only.

Each stage of the method comprises the steps that will now be briefly described. In their description reference is made to the use of DFO as a Fe chelator, but this should be construed as an example and not a limitation, as other Fe chelators may be used within the scope of the invention.
Step A - **Preparation of desferrioxamine (DFO) -** For the preparation and structure of DFO see e.g. H. Bickel et al, Helv. Chim Acta 43, 2129 (1960) and USP 3,471,476.
Step B - **Preparation of Fl-DFO -** This fluorescent probe is available from Molecular Probes Inc., Eugene by special order. The probe can be prepared by conjugating fluorescein-isothiocyanate (FITC) with DFO in a manner similar to that described for another fluorescent derivative of DFO, NBD-DFO (see Lytton et al., Mol. Pharmacol. 40,584 (1991 and Anal. Biochem. 205, 326 (1992).
Step C - **Preparation of a calibration curve.** Will be described hereinafter.
**Step D - Contacting the reagent A or B (Fl-DFO or Fl-DFO + DFO) with the sample -** The sample is placed in a number of well plates, preferably 96-well plates. To each well a reagent, which has been stored at low temperature and has been recently thawed; is added. The plates are incubated.
Step E - **Determination of the fluorescence** - The fluorescence in the wells is determined in a multiwell plate reader.
Step F - **Determination of the iron concentration -** The ratio between the fluorescence of the samples obtained with Reagents A (reading A) and B (reading B) is calculated, and the Fe concentration is derived by entering the said ratio A/B in the calibration curve.
If a mobilizer is to be added to the sample, it is included in both reagents A and B in stage D (see Example 2).

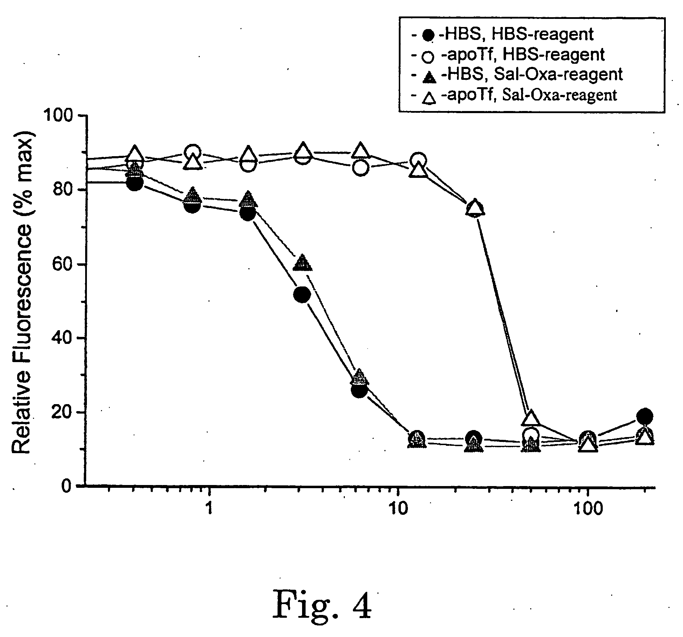

In the same way in which the aforesaid embodiment is illustrated in Fig. 1, a second, particularly preferred embodiment is illustrated in Fig. 4. It is clearly seen that the second embodiment differs from the first because F l-aTf is used in place of Fl-DFO. Gallium chloride is preferably added in this embodiment to the Fl-aTf probe, though this is not marked in Fig. 4. The procedure by which this embodiment is carried out is the same as summarized hereinbefore with reference to the first embodiment, with the difference that Fl-aTf is prepared, in the way that will be later described, instead of Fl-DFO.

The following examples illustrate and do not limit the invention.

Example 1 is an embodiment of the method of the invention, carried out without the use of mobilizing agents.

### Example 1

Samples of 20 µl of serum on which the NTBI assay is to be carried out ("input sample") and of Fe-free HBS (blank) are placed in quadruplicate in 96-well plates.
Two reagent solutions are prepared:
Reagent A: Fe-free HBS containing 2.5 µM Fluorescein-DFO (Fl-DFO).
HBS means a solution composed of 150 mM NaCl, 20 mM Hepes, pH 7.3; wherein Hepes means a commercially available pH buffering compound which is commonly used in biological solutions to maintain a stable pH.
All of the reagents must be used fresh, however they can be prepared complete, stored frozen at - 20°C in portions and thawed once. Repeated freeze-thaw is not recommended as it causes loss of fluorescence.
Reagent A (100 µl) is added to 2 of the wells, Reagent B is added to the 2 other wells.
The plates are incubated for 2 hrs at 37°C.
The fluorescence in the wells is determined in a multiwell plate reader (BMG LabTechnologies, Offenburg, Germany) with excitation/ emission filters of 485/ 538 nm and gain of 25.
The ratio between the fluorescence of the samples obtained with Reagents A (reading A) and B (reading B) is calculated, and the Fe concentration is derived from the calibration curve.

The choice of mobilizing agent mixtures should be based on two criteria:
maximum NTBI detection (viz. maximum Fl-DFO quenching activity) by a serum containing NTBI, and absence of release of Fe from Fe-containing transferrin. The preferred developed mobilizing agent mixture, used in the following Example 2, is composed of 15 mM sodium oxalate in HBS solution. However, other mobilizing agents or other combinations thereof could be used, as long as they cause significant Fl-DFO quenching in a sample which showed no Fl-DFO quenching in the absence of a mobilizing agent, and do not cause any significant quenching by samples known not to contain NTBI but to contain transferrin-bound iron, such as normal human sera or human transferrin saturated with various concentrations of Fe.

Example 2 is an embodiment of the method of the invention, carried out with the use of mobilizing agents.

### Example 2

This example includes the following steps:
Step 1 - Removal of apo-Tf (apo-transferrin, which means iron-free transferrin): Samples of 100 µl of serum are mixed with 800 µl of double distilled water and added to 500 µl of packed anionic beads (Macro-Prep® High S Support, obtained from Bio-Rad Laboratories, Hercules, CA). After 10 min of gentle mixing, the beads carrying bound apo-Tf are allowed to settle, and 150 µl of the supernatant solution is transferred into 4 wells. Alternatively, or additionally, apo-Tf and Tf can be removed from 100 µl of serum by addition of 100 µl of monoclonal anti-Tf antibodies coupled to either Sepharose beads (Pharmacia, Upssala) or Agarose beads (BioRad Laboratories, Hercules CA), incubating for 1hr and decantation of the beads.
Step 2: Two reagent solutions are prepared:
   Reagent A: 60 mM sodium oxalate, 300 mM NaCl, 80 mM Hepes, pH 7.3 and 5 µM Fluorescein-DFO (FI-DFO).
   Reagent B: Same as reagent A, but containing 100 µM DFO.
Step 3: Reagent A (50 µl) is added to 2 of the wells. Reagent B (50 µl) is added to the 2 other wells.
Step 4: Incubation at room temperature for 30 min.

The remaining steps of fluorescence determination, and calculation of the ratio of A/B are carried out as in Example 1. Construction of a calibration curve is carried out as in Example 3, but the samples are treated as described above.

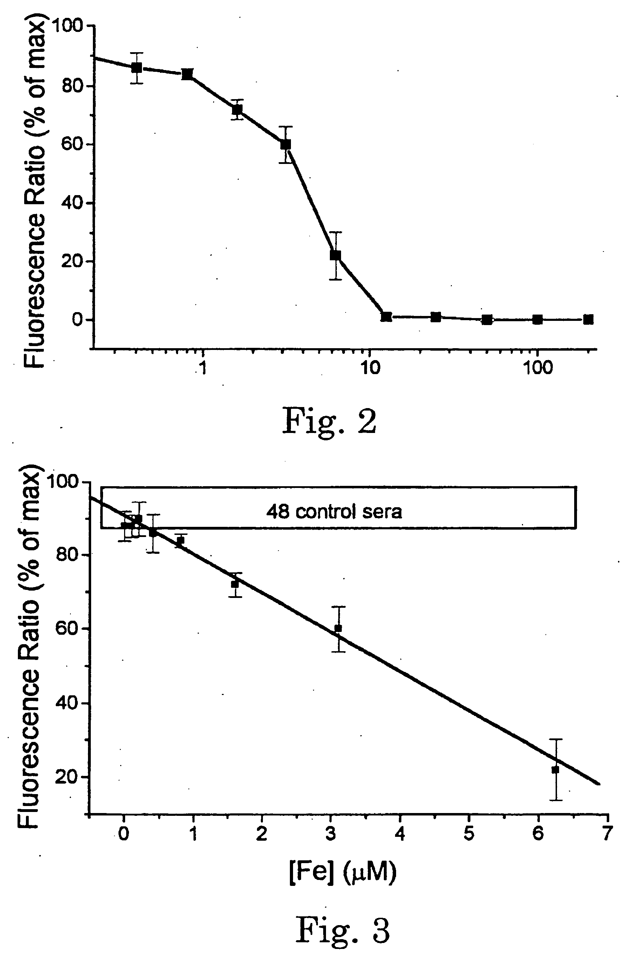

Example 3 illustrates, with reference to Figs. 2 and 3, the construction of a calibration curve.

### Example 3

To 0.1 ml of 10 mM ferrous ammonium sulfate, freshly prepared in double distilled water in a polyethylene tube, is added 0.1 ml of 70 mM nitrilotriacetate, sodium form, pH 7.0, to produce a Fe:NTA (5:35 mM) complex.
A serial 1:1 dilution of the Fe:NTA is performed in Fe-free HBS for up to 12 steps, to give Fe concentrations from 200 down to 0.2 µM.
Twenty µl of each Fe concentration are transferred in quadruplicate to 96-well plates and reagents A and B of Example 1 are added. The ratio of fluorescence of the plate samples generated by reagents A and B is plotted *versus* the input [Fe] (Fig. 2) and the linear portion (0-7 µM Fe) (Fig. 3) is used for calculating the serum [NTBI] values in the assayed samples.

The sensitivity of the assay was examined by preparing a series of Fe concentrations in HBS solution. The meaning of the various curves is marked on the side. The plateau of the curve in Fig. 2 shows that the iron binding capacity of the Fl-DFO reagent is saturated when the Fe concentration of the input sample is 12.5 µM. Taking into account that the 20 µl input sample of 12.5 µM Fe is diluted 6-fold by the addition of 100 µl of the 2.5 µM Fl-DFO reagent, the final concentrations of Fe and Fl-DFO are each approximately 2.1 µM. This 1:1 quenching ratio matches the predicted stoichiometry of Fl-DFO : Fe, since each DFO molecule binds one Fe. The linear and useful range of the HBS curves is that corresponding to Fe concentrations of 0 - 6.25 µM in the input sample. The upper and lower limits of detection are about 7 and 0.4 µM respectively.

Example 4 is an embodiment of the method of the invention, carried out by using F I-aTf as fluorescent probe.

Preparation of fluorescein-apo-Transferrin (Fl-aTf) To a solution containing 8 mg/ml apo-Transferrin (100 µM, based on MW 80,000 D) in 100 mM NaHCO₃, pH 8.4, was added 100 µM 5-(4,6-dichlorotriazinyl)aminofluorescein (DCTAF) from a freshly prepared 10 mM solution in dimethylsulfoxide. After incubation for 30 minutes at 37°C (protected from light), 5 mM L-lysine was added from a solution containing 0.5 M L-lysine, pH 8, in order to stop the coupling reaction. The Fl-aTf was exhaustively dialyzed against HBS, divided into aliquots and stored frozen at -20°C. The final preparation was presumed to contain 100 µM Fl-aTf and gave a single fluorescent band of approximately 80,000 D on polyacrylamide electrophoresis. The fluoroscein concentration in Fl-aTf was determined as 84 µM by absorbance at 496 nm, using DCTAF as standard solution, giving a ratio of aTf : fluoroscein of 1 : 0.84. Comparison of the Fe-induced quenching of Fl-aTf from three separate preparations showed them to be virtually identical.

### Preparation of solutions for the NTBI assay.

Two reagent solutions are prepared: Reagent A: HBS (150 mM NaCl, 20 mM Hepes, pH 7.3) containing 10 mM sodium oxalate, 0.1 mM GaCl₃ and 0.6 µM Fl-aTf. Reagent B is the same as reagent A, with the addition of 2 mg/ml aTf. Both reagents can be prepared complete, stored frozen at-20°C in portions and thawed just before use. Repeated freeze-thaw is avoided, due to its unknown effects on the reagents.

The concentrations of oxalate, Ga(III) and Fl-aTf were established empirically to give the maximum change in fluorescence in response to 0-6µM Fe and to generate the maximal NTBI values in serum samples known to contain NTBI, without giving rise to artifactual positive values in control sera.

### Measurement of serum NTBI.

The principle of the method is outlined in Fig. 4. Samples of 10 µl of serum (defined as "input sample") or HBS (blank) are placed in quadruplicate in 96-well plates. Reagent A (200 µl) is added to two of the wells, reagent B (200 µl) is added to the two other wells. The plates are incubated in the dark for one hour at room temperature. The fluorescence in the wells is determined in a multiwell plate reader (BMG LabTechnologies, Offenburg, Germany) with excitation/emission filters of 485/538 nm and gain of 25.

The ratio between the fluorescence of the samples obtained with reagents A (reading A) and B (reading B) is calculated, and the Fe concentration is derived from the calibration curve. In order to preclude the possibility of 'false-positive' results, we have set an arbitrary '0 value' for NTBI, based on measurements of 52 control sera. The '0 value' corresponds to 81% fluorescence ratio, equivalent to 1.5 µM Fe. This value (1.5 µM Fe) was subtracted from all NTBI measurements.

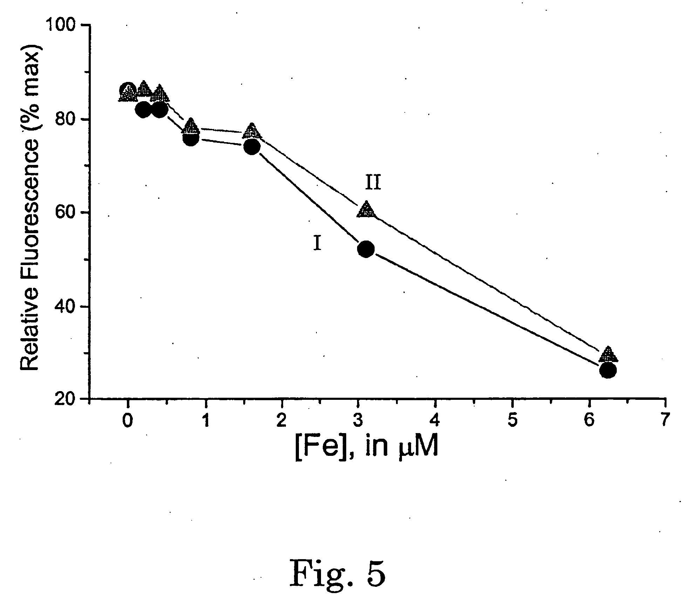

Fig. 5 illustrates the calibration of the iron concentration versus the fluorescence. A series of concentrations of iron (Fe-NTA form) was prepared in the HBS buffer (input sample). From each dilution, replicate 10 µl samples were transferred to 96-well plates followed by 200 µl of reagents 'A' and 'B'. The Fluorescence Ratio, expressed as "% of maximal value", reflects the ratio of fluorescence obtained in the absence and presence of excess aTf. [Fe] refers to the Fe concentration in the original 10 µl input sample. The linear region of the calibration curve (0-3.2 µM Fe) is shown. The value range labeled "52 control samples" represents an average ± S.D. for sera of 52 individuals without iron-overload. All serum samples with values below the designated "Arbitrary 0 value" (dotted line) are considered NTBI-positive. Error bars indicate S.D., n=3.

It will be apparent that the invention can be carried out by persons skilled in the art with many modifications, variations and adaptations, with respect to the examples, without exceeding the scope of the claims.

## Claims

1. A method of determining the content of non-transferrin-bound iron (NTBI) and/or chelator accessible iron in a sample, the method comprising:
(a) contacting
(i) a first aliquot of the sample with a fluorescent probe composed of a fluorescent marker combined with an Fe chelator; and
(ii) a second aliquot of the sample with said fluorescent probe and excess of said Fe chelator; and
(b) calculating ratio of fluorescence resultant from (i) and (ii), said ratio being indicative of content of non-transferrin-bound iron (NTBI) and/or chelator accessible iron in the sample.

2. The method of claim 1, wherein said fluorescent marker is selected from the group comprising fluorescein and a derivative of fluorescein.

3. The method of claim 1, wherein said Fe chelator is selected from the group consisting of desferrioxamine, hydroxypyridines, phenanthrolines and triazines.

4. The method of claim 1, wherein said fluorescent probe is fluoresceinated-deferrioxamine (FL-DFO).

5. The method of claim 1, wherein said fluorescent probe is fluorescein-labeled apo-Transferrin (Fl-aTf).

6. The method of claim 1, further comprising contacting both aliquots of said sample with a source of Gallium ions prior to (b).

7. The method of claim 1, wherein said first reagent and said second reagent further include an NTBI mobilizer.

8. The method of claim 7, wherein said NTBI mobilizer is sodium oxalate.

9. The method of claim 1, further comprising generating a calibration curve and deriving the Fe concentration of said sample using said calibration curve.

10. The method of claim 5, wherein preparing said fluorescein-labeled apo-Transferrin (Fl-aTf) is effected by reacting iron-free aTf with 5-(4,6-dichlorotrianzinyl aminofluorescein (DCTF).

11. A reagent for the measurement of non-transferrin bound iron comprising a fluorescent probe which comprises a fluorescent marker and an Fe chelator and Gallium ions.

12. The method according to claim 2, wherein said derivative of fluorescein is selected from the group consisting of coumarin and BODIPY.

## Patentansprüche

1. Verfahren zur Bestimmung des Gehalts von nicht-transferringebundenem Eisen (NTBI) und/oder chelatorzugänglichem Eisen in einer Probe, wobei das Verfahren umfasst:
(a) Inkontaktbringen
(i) einer erste Aliquote der Probe mit einer fluoreszierenden Sonde, zusammengesetzt aus einem fluoreszierenden Marker, kombiniert mit einem Fe-Chelator; und
(ii) einer zweiten Aliquote der Probe mit besagter fluoreszierender Sonde und Überschuss besagten Fe-Chelators; und
(b) Berechnens des aus (i) und (ii) resultierenden Fluoreszenzverhältnisses, wobei besagtes Verhältnis indikativ für den Gehalt an nicht-transferringebundenem Eisen (NTBI) und/oder chelatorzugänglichem Eisen in der Probe ist.

2. Verfahren von Anspruch 1, wobei besagter fluoreszierender Marker aus der Fluorescein und ein Derivat von Fluorescein umfassenden Gruppe gewählt ist.

3. Verfahren von Anspruch 1, wobei besagter Fe-Chelator aus der aus Desferrioxamin, Hydroxypyridinen, Phenanthrolinen und Triazinen bestehenden Gruppe gewählt ist.

4. Verfahren von Anspruch 1, wobei besagte fluoreszierende Sonde fluoresceiniertes Deferrioxamin (FL-DFO) ist.

5. Verfahren von Anspruch 1, wobei besagte fluoreszierende Sonde fluoresceinmarkiertes Apotransferrin (Fl-aTf) ist.

6. Verfahren von Anspruch 1, weiter das Inkontaktbringen beider Aliquote besagter Probe mit einer Quelle von Galliumionen vor (b) umfassend.

7. Verfahren von Anspruch 1, wobei besagtes erstes Reagens und besagtes zweites Reagens weiter einen NTBI-Mobilisator beinhalten.

8. Verfahren von Anspruch 1, wobei besagter NTBI-Mobilisator Natriumoxalat ist.

9. Verfahren von Anspruch 1, welches weiter das Erzeugen einer Eichkurve und Ableiten der Fe-Konzentration besagter Probe unter Verwendung besagter Eichkurve umfasst.

10. Verfahren von Anspruch 5, wobei das Herstellen besagten fluoresceinmarkierten Apotransferrins (Fl-aTf) durch Reagieren von eisenfreiem aTf mit 5-(4,6-Dichlorotrianzinylaminofluorescein) (DCTF) bewerkstelligt wird.

11. Reagens zur Messung von nicht-transferringebundenem Eisen, umfassend eine fluoreszierende Sonde, die einen fluoreszierenden Marker und einen Fe-Chelator und Galliumionen umfasst.

12. Verfahren gemäß Anspruch 2, wobei besagtes Fluoresceinderivat aus der aus Cumarin und BODIPY bestehenden Gruppe gewählt ist.

## Revendications

1. Procédé pour déterminer la teneur d'un échantillon en fer non lié à la transferrine (NTBI) et/ou en fer accessible à un chélateur, le procédé comprenant le fait de :
(a) mettre en contact
(i) une première quantité aliquote de l'échantillon avec une sonde fluorescente composée d'un marqueur fluorescent combiné à un chélateur du fer ; et
(ii) une deuxième quantité aliquote de l'échantillon avec ladite sonde fluorescente et un excès dudit chélateur du fer ; et
(b) calculer le rapport des fluorescences résultant de (i) et (ii), ledit rapport indiquant la teneur de l'échantillon en fer non lié à la transferrine (NTBI) et/ou en fer accessible à un chélateur.

2. Procédé selon la revendication 1, dans lequel ledit marqueur fluorescent est choisi parmi le groupe comprenant la fluorescéine et un dérivé de la fluorescéine.

3. Procédé selon la revendication 1, dans lequel ledit chélateur du fer est choisi parmi le groupe constitué par la desferrioxamine, des hydroxypyridines, des phénanthrolines et des triazines.

4. Procédé selon la revendication 1, dans lequel ladite sonde fluorescente est la desferrioxamine fluorescéinée (FL-DFO).

5. Procédé selon la revendication 2, dans lequel ladite sonde fluorescente est l'apo-transferrine marquée à la fluorescéine (Fl-aTf).

6. Procédé selon la revendication 1, comprenant en outre la mise en contact des deux quantités aliquotes dudit échantillon avec une source d'ions gallium avant de passer à l'étape (b).

7. Procédé selon la revendication 1, dans lequel ledit premier réactif et ledit deuxième réactif englobent en outre un mobilisateur du NTBI.

8. Procédé selon la revendication 7, dans lequel ledit mobilisateur du NTBI est l'oxalate de sodium.

9. Procédé selon la revendication 3, comprenant en outre le fait de générer une courbe d'étalonnage et le fait de dériver la concentration de Fe dudit échantillon en utilisant ladite courbe d'étalonnage.

10. Procédé selon la revendication 5, dans lequel la préparation de ladite apo-transferrine marquée à la fluorescéine (Fl-aTf) est mise en oeuvre en faisant réagir de la aTf exempte de fer avec de la 5-(4,6-dichlorotriazinylamino)fluorescéine (DCTF).

11. Réactif pour la mesure de la teneur en fer non lié à la transferrine, comprenant une sonde fluorescente qui comprend un marqueur fluorescent et un chélateur du fer, ainsi que des ions gallium.

12. Procédé selon la revendication 2, dans lequel ledit dérivé de la fluorescéine est choisi parmi le groupe constitué par la coumarine et par BODIPY.
